# EUROPEAN PATENT APPLICATION

(11) **EP 1 884 570 A1**
(43) Date of publication of application: **06.02.2008**
(21) Application number: 05743756.8
(22) Date of filing: 26.05.2005
(51) Int. Cl.: C12N 15/87, A61K 48/00, A61N 7/00

(54) **GENE TRANSFER METHOD**

(71) Applicant: Mebiopharm Co., Ltd., Tokyo 107-0052 (JP)
(72) Inventor: MARUYAMA, Kazuo, Sagamihara-shi, Kanagawa 2291101 (JP); TAKIZAWA, Tomoko, Sagamihara-shi, Kanagawa 2200202 (JP); HAGISAWA, Kosuke, Sapporo-shi, Hokkaido 062-0932 (JP); NISHIOKA, Toshihiko, Saitama-shi, Saitama 331-0812 (JP); YANAGIE, Hironobu, Tokyo 1060032 (JP)
(74) Representative: Hartz, Nikolai
(86) International application number: PCT/JP2005/009637
(87) International publication number: WO 2006/126267

(57) **Abstract**

A method for efficiently transferring a gene to a target cell is provided. A method of transferring a gene to a target cell, including adding or administering a positively charged complex (A) composed of the gene and a cationic substance and gas-filled microparticles (B) to a target cell-containing composition or a living body and then exposing the target cell-containing composition or the living body to a low-frequency ultrasound.

## Description

### Technical Field

The present invention relates to a method of and kit for efficiently transferring a gene to a target cell in in vivo and in vitro.

### Background Art

As methods of transferring a gene to a target cell, for example, a method of administering a gene enclosed in quaternary ammonium salt-containing liposomes (Non-Patent Document 1) and a method of administering a gene in conjunction with protamine or the like (Non-Patent, Document 2) are known. However, these methods are not satisfactory yet in their transfer efficiency of a gene to a target cell.

In addition, it is known that a gene can be transferred to a target cell by administering the gene simultaneously with microbubbles made of a thin shell of albumin enclosing a propane octafluoride gas or the like therein and exposing the microbubbles to an ultrasound to cause cavitation of the enclosed gas (Non-Patent Document 3).
[Non-Patent Document 1] Felgner, P. L. Cationic liposome-mediated transfection with lipofection reagent. Meth. Mol. Biol. 1991, 91-98.
[Non-Patent Document 2] Gao, X. and Huang, L., A novel cationic liposome reagent for efficient transfection of mammalian cells. Biochem. Biophys. Res. Commun. 1991, 179, 280-285.
[Non-Patent Document 3] Tachibana, K., Uchida, T., Ogawa, K., Yamashita, N., Tamura, K., Induction of cell-membrane porosity by ultrasound. Lancet 1999, 353, 1409,

### Disclosure of the Invention

### Problems to be Solved by the Invention

However, the gene transfer efficiency with the above method using microbubbles is still low. Consequently, a method that can achieve higher transfer efficiency has been desired.

### Means for Solving the Problems

Accordingly, the present inventors have completed the present invention by arriving at the fact that the transfer efficiency of a gene to a target cell can be dramatically improved by 10 to 10000 times of those of conventional methods by previously combining the gene and a cationic substance into a complex having a positive surface charge and exposing this complex in conjunction with microbubbles to an ultrasound, instead of using the gene and the microbubbles as they are.

That is, the present invention provides a method of transferring a gene to a target cell, including adding or administering a positively charged complex (A) composed of the gene and a cationic substance and gas-filled microparticles (B) to a target cell-containing composition or a living body, and then exposing the target cell-containing composition or the living body to a low-frequency ultrasound.
The present invention further provides a kit for transferring a gene to a target cell, wherein the kit including a positively charged complex (A) composed of the gene and a cationic substance, and gas-filled microparticles (B).

### Effect of the Invention

According to the present invention, an objective gene can be transferred to a target cell with significantly high efficiency in both in vitro and in vivo. Therefore, the present invention can increase the production ratio of transformed cells that can not been obtained by conventional methods due to their low transfer efficiency. Furthermore, the present invention can dramatically increase the efficacy ratio of gene therapy.

### Best Mode for Carrying Out the Invention

The present invention is characterized by using a positively charged complex (A) of a gene and a cationic substance. Here, examples of the gene include DNAs, RNAs, antisense DNAs, siRNAs, decoys, and therapeutic oligonucleotides. Examples of the cationic substance include cationic peptides such as protamine, poly-L-lysine, poly-L-arginine, and ornithine; and cationic polymers such as polyethyleneimine, cationic dendrimers, and chitosan. The complex of the gene and the cationic substance can be prepared, for example, by mixing the gene and the cationic substance in purified water. Since aggregation may occur depending on the solvent, a previous examination should be performed. In addition, it is necessary that the entire charge of the prepared complex is positive. The charge is preferably adjusted to +5 to +20 mV as the zeta potential. The zeta potential can be measured with a commonly-used zeta potential analyzer.

The particle diameter of the complex is preferably about 100 to 300 nm from the viewpoint of gene transfer efficiency. This particle diameter can be measured with a laser scattering particle analyzer.

The gene and the cationic substance to be used are preferably mixed at a weight ratio of 1:100 to 100:1 and more preferably at a ratio of 1:10 to 10:1.

In addition, as the gas-filled microparticles (B), conventionally used microbubbles can be used, for example, such as albumin microspheres enclosing a gas therein and gas-filled liposomes. Examples of known microbubbles include Alubunex (Molecular Biosystems), Levovist (Schering), Sonavist (Schering), Echovist (Schering), Sonazoid (Nycomed), Optison (Nycomed-Amersham), Definity (DuPont Pharmaceutical), and SonoVue (Bracco).

Examples of the gas-filled liposomes include gas-filled liposomes that are prepared by filling the void space of a sealed container containing a liposome suspension in a volume amounting to 20 to 80% of the inner capacity thereof with a fluoride gas or a nitrogen gas and then exposing them to an ultrasound.

Examples of lipids used as the membrane constituent of the liposome include phospholipids, glyceroglycolipids, sphingoglycolipids, cationic lipids in which a primary amino group, a secondary amino group, a tertiary amino group, or a quaternary ammonium group is introduced into the above lipids, lipids in which polyalkylene glycols are introduced into the above lipids, and lipids to which ligands to various types of cells, tissues and the like are bound.

The phospholipids includes natural and synthetic phospholipids, such as phosphatidylcholines (e. g., soybean phosphatidylcholine, egg yolk phosphatidylcholine, distearoyl phosphatidylcholine, and dipalmitoyl phosphatidylcholine), phosphatidylethanolamines (e. g., distearoyl phosphatidylethanolamine), phosphatidylserines, phosphatidic acid, phosphatidylglycerols, phosphatidylinositols, lysophosphatidylcholines, sphingomyelins, egg yolk lecithins, soybean lecithins, and hydrogen added phospholipids.

Examples of the glyceroglycolipids include sulfoxyribosyl glycerides, diglycosyl diglycerides, digalactosyl diglycerides, galactosyl diglycerides, and glycosyl diglycerides. Examples of the sphingoglycolipids include galactosyl cerebrosides, lactosyl cerebrosides, and gangliosides.

Examples of the cationic lipids include lipids in which an amino group, an alkylamino group, a dialkylamino group, or a quaternary ammonium group such as a trialkylammonium group, a monoacyloxyalkyldialkylammonium group or a diacyloxyalkylmonoalkylammonium group, is introduced into the above phospholipids, glyceroglycolipids or sphingoglycolipids. Examples of the polyalkylene glycol-modified lipids include lipids in which the above phospholipids, glyceroglycolipids or sphingoglycolipids are modified with polyethylene glycol, polypropylene glycol or the like such as di-C₁₂₋₂₄acyl-glycerolphosphatidylethanolamine-N-PEG.

In addition, a membrane stabilizer such as cholesterols and an antioxidant such as tocopherol, stearylamine, dicetylphosphate or ganglioside may be used, according to necessity.

Examples of the ligand to a target cell, a target tissue or a target lesion include ligands to cancer cells, such as transferrin, folic acid, hyaluronic acid, galactose and mannose. In addition, monoclonal antibodies and polyclonal antibodies can be used as the ligand.

The previously prepared liposomes may contain a gene or the like therein, as long as they have an aqueous phase in the inside.

The liposomes can be produced by a known process for preparing liposomes, for example, by the liposome preparation method of Bangham, et al., (J. Mol. Biol. 1965, 13, 238), an ethanol injection method (J. Cell. Biol. 1975, 66, 621), a French press method (FEBS Lett. 1979, 99, 210), a freeze and thawing method (Arch. Biochem. Biophys. 1981, 212, 186), or a reverse phase evaporation method (Proc. Natl. Acad. Sci. USA 1978, 75, 4194). For example, a liposome suspension is prepared by dissolving a lipid in an organic solvent, adding an aqueous solution thereto, and then treating the resulting mixture with an ultrasound. Then, if necessary, the suspension is applied to an extruder and/or a membrane filter for particle sizing. In such a case, the particles are preferably sized to have a particle diameter of 1 µm or less, more preferably 100 to 800 nm, and particularly preferably 100 to 600 nm.

The prepared liposome suspension is poured in a sealed container. In this stage, the void space of the container is preferably 20 to 80%, more preferably 30 to 80%, and particularly preferably 50 to 80% of the inner capacity of the container. When the void space is less than 20%, the induction ratio of a gas into the produced liposomes is too low. The void space exceeding 80% is uneconomical.

This void space is filled with a fluoride gas or a nitrogen gas. Examples of the fluoride gas include sulfur hexafluoride and perfluorohydrocarbon gases, such as CF₄, C₂F₆, C₃F_{8,} C₄F₁₀, C₅F₁₂, and C₆F₁₄. Among them, C₃F₈, C₄F₁₀, and C₅F₁₂ are particularly preferred. In addition, a nitrogen gas can also be used. The pressure of the filled gas is preferably 1 atmosphere (gauge pressure) or more and particularly preferably 1 to 1.5 atmospheres. A simple way for filling the void space with a gas is injection, for example, with a needle syringe through a rubber stopper. An injection cylinder may also be used.

Subsequently, an ultrasound treatment is conducted. For example, the container may be exposed to an ultrasound of 20 to 50 kHz for 1 to 5 minutes. With this ultrasound treatment, the aqueous solution in the liposomes is replaced with a fluoride gas or a nitrogen gas to give gas-filled liposomes. The given gas-filled liposomes have a particle diameter approximately the same as that of the raw liposomes. Accordingly, the gas-filled liposomes having a particle diameter within a certain range, e.g., 1 µm or less, more preferably 50 to 800 nm, and particularly preferably 100 to 600 nm, can be readily produced by sizing the raw liposomes when they are prepared.

Furthermore, the gas-filled liposomes can be readily produced at a site, such as a hospital, only by conducting an ultrasound treatment, if a sealed container containing a liposome suspension and filled with a fluoride gas or a nitrogen gas is previously prepared and supplied to the hospital or the like.

The gas-filled liposomes thus obtained can have a small particle diameter and a constant particle size distribution, and can be delivered to a microvasculature, a deep tissue or the like.

Furthermore, in the present invention, the above complex (A) may be enclosed in the gas-filled microparticles (B). The process for enclosing the complex into the microparticles may be conducted during the step of preparing the gas-filled microparticles, or may be performed after the preparation of the gas-filled microparticles by adding the complex (A) to the microparticles and mixing them.

In the present invention, the above complex (A) and the gas-filled microparticles (B) are added or administered to a target cell-containing composition or a living body. Examples of the target cell-containing composition include target cell culture solutions. Examples of the living body include mammals including human, birds, fishes, reptiles, insects, and plants. The target cell includes a cell into which a gene is introduced or a tissue including such a cell.

In a case of in vitro, the above complex (A) and the gas-filled microparticles (B) are added to a target cell culture solution and the mixture is exposed to a low-frequency ultrasound. In a case of in vivo, the above complex and the gas-filled microparticles are administered to a living body, followed by exposing the living body to diagnostic ultrasound (2 to 6 MHz) to confirm the delivery of the complex and the microparticles to the target cells. Once the delivery is confirmed, a low-frequency ultrasonication is conducted. The administration may be topical administration or intravenous administration.

Exposing the gas-filled microparticles (B) to a low-frequency ultrasound containing a resonance frequency of 0.5 to 2 MHz leads to disruption of the microparticles and cavitation caused by microbubbles of the gas. As a result, the above complex (A) or the above complex (A) in the gas-filled microparticles present near the cavitation site is efficiently introduced into the target cells. The mechanism that the complex (A) is efficiently induced into the target cells is unclear, but is assumed that the complex can be readily brought into contact with the cell surfaces due to its positive charge.

### Example

The present invention will hereinafter be described in detail with reference to the example, but is not limited thereto.
Abbreviations used in the example are as follows:
DPPC: dipalmitoyl phosphatidylcholine
DOPE: dioleoyl phosphatidylethanolamine
DOTAP: 1,2-diolsoyl-3-trimethylammonium-propane

### Example 1

(1) Plasmid DNAs coding luciferase were mixed with protamine to prepare a DNA-protamine complex followed by reducing the size thereof. The entire charge of the complex was adjusted to be positive (+0.5 to +20 mV of zeta potential). For comparison, the complex having negative entire charge (-7 mV of zeta potential) was also made.

(2) DPPC liposome
   Lipids of DPPC and cholesterol (1:1, (m/m)) were dissolved in an organic solvent mixture of chloroform and isopropyl ether (1:1, v/v), and an aqueous solution such as saline (or an aqueous solution containing a drug) was added thereto in a volume amounting to a half of the organic solvent (i.e., chloroform : isopropyl ether : aqueous solution = 1:1:1, v/v). The resulting mixture was mixed to give an emulsion. The emulsion was subjected to a reverse phase evaporation method (REV method) to prepare liposomes. The liposomes were sized by passing them through polycarbonate membranes of 400 nm, 200 nm and 100 nm with an extruder.

(3) DOTAP liposome
   DOTAP and DOPE (1:1, (w/w)) were dissolved in chloroform, and the mixture was put in a pear-shaped flask. The organic solvent was evaporated while rotating with a rotary evaporator to produce a thin film of a lipid on the wall (production of a lipid film). Then, hydration was conducted using a solvent such as saline to produce liposomes. The liposomes were reduced in size by an ultrasound treatment or by passing them through polycarbonate membranes of 400 nm, 200 nm and 100 nm with an extruder.

(4) The following reagents were used as commercially available gene-delivering reagents composed of cationic liposomes:
   Lipofectin^{™} (DOTMA : DOPE = 1:1, w/w), and
   LipofectACE^{™} (DDAB : DOPE = 1:1.25, w/w).

(5) Enclosure of perfluoropropane gas
   A liposome aqueous solution (lipid concentration: 5 mg/mL) was put in a vial (5 mL, 10 mL, or 20 mL, for example) in a volume amounting to 30% of the capacity of the vial (1.5 mL, 3 mL, or 6 mL). Perfluoropropane gas was put into the vial to replace for air therein. The vial was sealed with a rubber stopper, and perfluoropropane was further added through the rubber stopper with a needle syringe to the volume of 1.5 times of the inner capacity, so that the inner pressure became about 1.5 atms. A bath-type ultrasound apparatus (42 kHz) was filled with water, and the vial was left standing therein and exposed to an ultrasound for one minute.

(6) AsPC-1 cells (4 × 10⁴ cells/well) were cultured in a 48-well plate. The DNA-protamine complex (1 µg of DNA, lipid : DNA = 12:1, w/w) and the gas-filled PEG-liposomes were added thereto and then exposed to a pulsed ultrasound of 1 MHz for three seconds. The culture solution was immediately washed three or four times repeatedly. After addition of a culture medium, the cells were further cultured for two days. Then, luciferase activity was measured by a conventional method. The results are shown in Table 1.

**[Table 1]**

| Perfluoropropane gas-filled liposome | Charge state of DNA/protamine complex | Ultrasound treatment | Luciferase activity (RLU/mg protein) |
|---|---|---|---|
| DPPC LP | positive charge | | 0.6×10³ |
| DPPC LP | positive charge | +SONIC | 6.3×10³ |
| DPPC LP | negative charge | | 0.1×10³ |
| DOTAP LP | positive charge | | 4.9×10⁶ |
| DOTAP LP | positive charge | +SONIC | 205×10⁶ |
| DOTAP LP | negative charge | | 0.1×10⁶ |
| Lipofectin^{™} | positive charge | | 0.1×10⁶ |
| Lipofectin^{™} | positive charge | +SONIC | 129×10⁶ |
| LipofectACE^{™} | positive charge | | 0.3×10⁶ |
| LipofectACE^{™} | positive charge | +SONIC | 135×10⁶ |

It was indicated from the results that the high expression level was achieved when perfluoropropane gas-filled cationic liposomes and the positively charged DNA/protamine complex were exposed to a low-frequency ultrasound.

## Claims

1. A method of transferring a gene to a target cell, comprising adding or administering a positively charged complex (A) composed of the gene and a cationic substance and gas-filled microparticles (B) to a target cell-containing composition or a living body, and then exposing the target cell-containing composition or the living body to a low-frequency ultrasound.

2. The method of transferring a gene according to Claim 1, wherein the target cell-containing composition is a target cell culture solution.

3. The method of transferring a gene according to Claim 1 or 2, wherein the cationic substance is a cationic peptide or a cationic polymer.

4. The method of transferring a gene according to any one of Claims 1 to 3, wherein the gas-filled microparticles are microspheres of a polymer or liposome enclosing a gas therein.

5. The method of transferring a gene according to any one of Claims 1 to 4, wherein the positively charged complex (A) composed of the gene and a cationic substance is enclosed in the gas-filled microparticles (B).

6. A kit for transferring a gene to a target cell, comprising a positively charged complex (A) composed of the gene and a cationic substance, and gas-filled microparticles (B).

7. The kit according to Claim 6, wherein the kit is used for adding the positively charged complex (A) composed of a gene and a cationic substance, and gas-filled microparticles (B) to a target cell-containing composition and then exposing the target cell-containing composition to a low-frequency ultrasound.

8. The kit according to Claim 6, wherein the kit is used for administering the positively charged complex (A) composed of a gene and a cationic substance and gas-filled microparticles (B) to a living body and then exposing the living body to a low-frequency ultrasound.

9. The kit according to Claim 6 or 8, wherein the target cell-containing composition is a target cell culture solution.

10. The kit according to any one of Claims 6 to 9, wherein the cationic substance is a cationic peptide or a cationic polymer.

11. The kit according to any one of Claims 6 to 9, wherein the gas-containing microparticles are microspheres of a polymer or liposome enclosing a gas therein.

12. The kit according to any one of Claims 6 to 11, wherein the positively charged complex (A) composed of a gene and a cationic substance is enclosed in the gas-filled microparticles (B).
